(19) Europäisches Patentamt
European Patent Office
Office européen des brevets

(11) Veröffentlichungsnummer: **0 044 527**
**A1**

(12) **EUROPÄISCHE PATENTANMELDUNG**

(21) Anmeldenummer: **81105575.5**

(22) Anmeldetag: **15.07.81**

(51) Int. Cl.³: **C 07 H 19/20**
**A 61 K 31/70**

(30) Priorität: **18.07.80 DE 3027279**

(43) Veröffentlichungstag der Anmeldung:
**27.01.82 Patentblatt 82/4**

(84) Benannte Vertragsstaaten:
**BE IT**

(71) Anmelder: **Byk Gulden Lomberg Chemische Fabrik GmbH**
**Byk-Gulden-Strasse 2**
**D-7750 Konstanz(DE)**

(72) Erfinder: **Engels, Joachim, Dr.**
**Department of Chemistry University of Colorado**
**Boulder, Colorado 80309(US)**

(54) **Substituierte Adenosin-3',5'-phosphorsäurebenzyltriester, Verfahren zu ihrer Herstellung und diese Verbindungen enthaltende Arzneimittel.**

(57) Substituierte Adenosin-3',5'-phosphorsäurebenzyltriester der allgemeinen Formel I

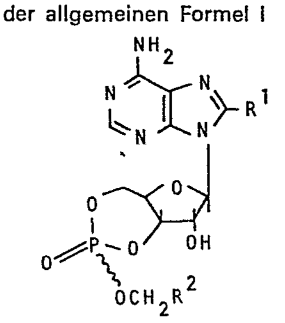

I,

wobei $R^1$ die Bedeutung Halogen, $SR^3$, worin $R^3$ einen geradkettigen oder verzweigten $C_1$-$C_5$-Alkyl- oder $C_2$-$C_5$-Alkenylrest, die durch Hydroxy oder Phenyl, das mit Halogen, Methyl, Ethyl, Methoxy, Ethoxy oder Nitro substituiert sein kann, substituiert sein können, einen $C_4$-$C_7$-Cycloalkylrest, Phenyl oder Naphthyl, die mit Halogen, Methyl, Ethyl, Methoxy oder Ethoxy ringsubstituiert sein können, bedeutet, $SeR^4$, worin $R^4$ einen geradkettigen oder verzweigten $C_1$-$C_5$-Alkylrest, oder Benzyl, das mit Halogen, Methyl, Ethyl, Methoxy, Ethoxy oder Nitro ringsubstituiert sein kann, bedeutet, oder $NR^5R^6$, worin $R^5$ und $R^6$, die gleich oder verschieden sind, Wasserstoff oder $R^3$ bedeuten, oder zusammen mit dem Stickstoffatom einen Piperidino- oder Morpholinorest darstellen, und $R^2$ die Bedeutung Phenyl, das durch elektronenliefernde oder elektronenziehende Gruppen substituiert sein kann, haben, sind neu and zeigen interessante pharmakologische Wirkungen, wie z.B. inotrope Wirkung, relaxierende Wirkung auf den Respirationstrakt und die Blutgefäße. Ihre Herstellung und Verarbeitung zu Arzneimitteln werden beschrieben.

EP 0 044 527 A1

Substituierte Adenosin-3',5'-phosphorsäurebenzyltriester, Verfahren zu ihrer Herstellung und diese Verbindungen enthaltende Arzneimittel

## Technisches Gebiet

Die Erfindung betrifft substituierte Adenosin-3',5'-phosphorsäurebenzyltriester, ein Verfahren zu ihrer Herstellung und diese Verbindungen enthaltende Arzneimittel.

## Stand der Technik

Bekannt sind Adenosin-3',5'-phosphorsäurebenzylester (cAMP-Benzylester, Naunyn-Schmiedeberg's Arch. Pharmacol. 310, 103-111 [1979]) und 8-substituierte cAMP-Derivate (J.Med.Chem. 1980, 23 242-251). Bekannt sind auch deren positiv inotrope Wirkungen an isoliertem Meerschweinchen-Myokard. Es hat sich jedoch bei diesen Verbindungen gezeigt, daß sie ihre Wirkungen erst bei relativ hohen Konzentrationen zeigen, d.h. im millimolaren Bereich, was für die systemische Anwendung häufig bereits uninteressant ist. In einigen Fällen zeigten sich auch negative Nebenwirkungen, so wurden beispielsweise beim Dibutyryl-cAMP anfangs negativ inotrope Wirkungen festgestellt.

Aufgabe der Erfindung ist es daher, neue Stoffe zu finden, die in geringerer Konzentration die erwünschte Wirkung zeigen und möglichst frei von Nebenwirkungen sind.

## Beschreibung der Erfindung

Gegenstand der Erfindung sind substituierte Adenosin-3',5'-phosphorsäurebenzyltriester der allgemeinen Formel

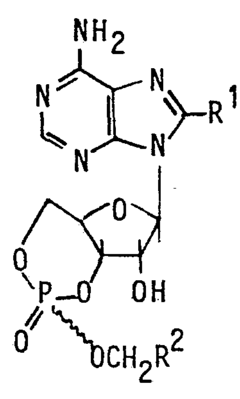

I,

wobei

$R^1$ die Bedeutung Halogen, $SR^3$, worin $R^3$ einen geradkettigen oder verzweigten $C_1$-$C_5$-Alkyl- oder $C_2$-$C_5$-Alkenylrest, die durch Hydroxy oder Phenyl, das mit Halogen, Methyl, Ethyl, Methoxy, Ethoxy oder Nitro substituiert sein kann, substituiert sein können, einen $C_4$-$C_7$-Cycloalkylrest, Phenyl oder Naphthyl, die mit Halogen, Methyl, Ethyl, Methoxy oder Ethoxy ringsubstituiert sein können, bedeutet, $SeR^4$, worin $R^4$ einen geradkettigen oder verzweigten $C_1$-$C_5$-Alkylrest, oder Benzyl, das mit Halogen, Methyl, Ethyl, Methoxy, Ethoxy oder Nitro ringsubstituiert sein kann, bedeutet, oder $NR^5R^6$, worin $R^5$ und $R^6$, die gleich oder verschieden sind, Wasserstoff oder $R^3$ bedeuten, oder zusammen mit dem Stickstoffatom einen Piperidino- oder Morpholinorest darstellen, und

$R^2$ die Bedeutung Phenyl, das durch elektronenliefernde oder elektronenziehende Gruppen substituiert sein kann, haben.

Ein bevorzugter Gegenstand der Erfindung sind substituierte Adenosin-3',5'-phosphorsäurebenzyltriester der allgemeinen Formel I'

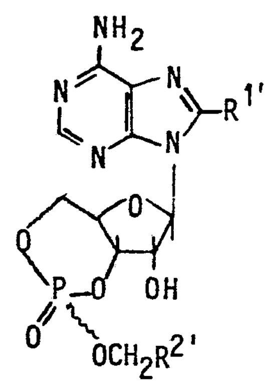

I',

wobei

$R^{1'}$ die Bedeutung Chlor, Brom, Iod, Methylthio, Ethylthio, t-Butylthio, Cyclohexylthio, Benzylthio, wobei die Phenylgruppe durch Halogen, Methyl oder Ethyl monosubstituiert sein kann, Phenylthio, wobei die Phenylgruppe durch Halogen, Methyl oder Ethyl monosubstituiert sein kann, Benzylseleno, wobei die Phenylgruppe durch Halogen, Methyl oder Ethyl monosubstituiert sein kann, $NR^{5'}R^{6'}$, wobei $R^{5'}$, $R^{6'}$ gleich oder verschieden sind und Wasserstoff, Methyl, Ethyl oder Benzyl bedeuten, und

$R^{2'}$ die Bedeutung Phenyl, wobei die Phenylgruppe durch Halogen, Methyl, Methoxy, Cyan oder Nitro einfach oder mehrfach substituiert sein kann,

haben.

Ein besonders bevorzugter Gegenstand der Erfindung sind substituierte Adenosin-3',5'-phosphorsäurebenzyltriester der allgemeinen Formel I"

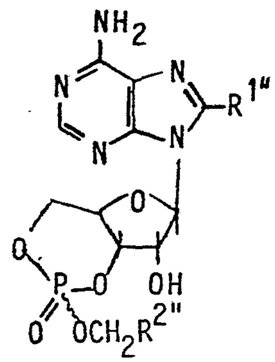

I",

wobei

$R^{1"}$ die Bedeutung Brom, Methylthio, t-Butylthio, Cyclohexylthio, Benzylthio, p-Chlorphenylthio, Benzylseleno, Benzylamino, Benzylmethylamino, und

$R^{2"}$ die Bedeutung Phenyl, p-Cyanophenyl und p-Methylphenyl haben.

Bevorzugte Verbindungen sind:
8-Brom-adenosin-3',5'-monophosphorsäurebenzylester;
8-Methylthio-adenosin-3',5'-monophosphorsäurebenzylester;
8-(4-Chlor-phenylthio)-adenosin-3',5'-monophosphorsäurebenzylester;
8-Cyclohexylthio-adenosin-3',5'-monophosphorsäurebenzylester;
8-t-Butylthio-adenosin-3',5'-monophosphorsäurebenzylester;
8-Benzylamino-adenosin-3',5'-monophosphorsäurebenzylester;
8-N-Benzyl-N-methylamino-adenosin-3',5'-monophosphorsäurebenzylester und
8-Benzylseleno-adenosin-3',5'-monophosphorsäurebenzylester;

Die erfindungsgemäßen Verbindungen werden hergestellt, indem die entsprechenden in 8-Stellung substituierten Adenosin-3',5'-monophosphorsäuren nach dem in J.Med.Chem. 1977, 20, 907-911 angegebenen Verfahren mit den entsprechenden Diazomethanen verestert werden. Die in 8-Stellung substituierten Adenosin-3',5'-monophosphorsäuren werden durch Halogenierung, vorzugsweise Bromierung, von Adenosin-3',5'-monophosphorsäure zu 8-Halogen-, vorzugsweise 8-Brom-adenosin-3',5'-monophosphorsäure hergestellt

und gegebenenfalls durch nucleophile Substitution des Halogenatoms analog zu dem in Biochemistry 10, 2390 (1971) angegebenen Verfahren in die erfindungsgemäßen Verbindungen überführt.

Ein weiterer Gegenstand der Erfindung ist ein Verfahren zur Herstellung der erfindungsgemäßen Verbindungen, das dadurch gekennzeichnet ist, daß eine Verbindung der allgemeinen Formel 8-$R^1$-cAMP, 8-$R^{1'}$-cAMP bzw. 8-$R^{1''}$-cAMP, worin $R^1$, $R^{1'}$ bzw. $R^{1''}$ die oben angegebenen Bedeutungen haben und cAMP Adenosin-3',5'-monophosphorsäure bedeutet, mit einem Diazomethanderivat der allgemeinen Formel $R^2CHN_2$, $R^{2'}CHN_2$ bzw. $R^{2''}CHN_2$, worin $R^2$, $R^{2'}$ bzw. $R^{2''}$ die oben angegebenen Bedeutungen haben, verestert wird.

Als besonders vorteilhaft hat es sich dabei erwiesen, die Veresterung in Hexamethylphosphorsäuretriamid (HMPT) durchzuführen, zum einen wegen einer guten Löslichkeit in diesem Lösungsmittel, zum anderen, weil darin das eingesetzte Diazomethan besonders stabil ist, und schließlich, weil in diesem System gute Ausbeuten erzielt werden. Die Veresterung in HMPT ist daher bevorzugt.

Die erfindungsgemäßen Verbindungen fallen dabei als Gemische an, deren beide Komponenten sich dadurch unterscheiden, daß die Estergruppe im Dioxaphosphorinanring einmal axial und einmal equatorial ausgerichtet ist. Um dies anzudeuten, ist in den allgemeinen Formeln I, I' und I" die Bindung zwischen dem Phosphoratom und dem Sauerstoffatom der Benzyloxygruppe als Wellenlinie dargestellt. Die Trennung der Diastereomeren ist auf chromatographischem Weg ohne weiteres möglich. Da sich die Diastereomeren in ihrer pharmakologischen Wirksamkeit nicht merklich unterscheiden, ist eine Auftrennung für die Verwendung als Arzneimittel nicht erforderlich.

Schließlich sind Gegenstand der Erfindung Arzneimittel, die sich durch einen Gehalt an einer oder mehreren der erfindungsgemäßen Verbindungen auszeichnen sowie die Anwendung der erfindungsgemäßen Verbindungen in der Behandlung von Erkrankungen des Herzens und/oder des Respirationstraktes. Oberraschenderweise wurde ferner gefunden, daß Arzneimittel,

die außerdem eine Phosphodiesterase hemmende Substanz enthalten, eine potenzierte Wirkung zeigen, die etwa beim 30- bis 40-fachen der Arzneimittel ohne diese zusätzliche Substanz liegt. Vorzugsweise wird als solche Substanz 1-Methyl-3-isobutylxanthin, Theophyllin und/oder Papaverin eingesetzt.

Ein weiterer Gegenstand der Erfindung sind daher Arzneimittel, die neben einer oder mehreren erfindungsgemäßen Verbindungen eine die Phosphodiesterase hemmende Substanz, vorzugsweise 1-Methyl-3-isobutylxanthin, Theophyllin und/oder Papaverin enthalten.

Die Arzneimittel werden nach an sich bekannten Verfahren hergestellt, wobei die neuen Verbindungen bzw. deren Kombinationen mit Phosphodiesterasehemmern als solche oder gegebenenfalls in Kombination mit geeigneten pharmazeutischen Trägerstoffen eingesetzt werden. Enthalten die neuen pharmazeutischen Zubereitungen neben den Wirkstoffen pharmazeutische Trägerstoffe, beträgt der Wirkstoffgehalt dieser Mischungen 0,5 bis 95, vorzugsweise 15 bis 75 Gewischtsprozent der Gesamtmischung.

In Übereinstimmung mit der Erfindung werden die Wirkstoffe in jeder geeigneten Formulierung angewandt unter der Voraussetzung, daß die Ausbildung bzw. Aufrechterhaltung von ausreichenden Wirkstoffspiegeln gewährleistet ist. Das kann beispielsweise durch orale, rektale oder parenterale Gabe in geeigneten Dosen erreicht werden. Üblicherweise liegt die pharmazeutische Zubereitung des Wirkstoffs in Form von Einheitsdosen vor, die auf die gewünschte Verabreichung abgestimmt sind. Eine Einheitsdosis kann zum Beispiel eine Tablette, ein Dragée, eine Kapsel, ein Suppositorium oder eine gemessene Volumenmenge eines Pulvers, eines Granulats, einer Lösung, einer Emulsion oder einer Suspension sein.

Unter "Einheitsdosis" im Sinne der vorliegenden Erfindung wird eine physikalisch bestimmte Einheit, die eine individuelle Menge des aktiven Bestandteils in Kombination mit einem pharmazeutischen Trägerstoff enthält, verstanden, deren Wirkstoffgehalt einem Bruchteil oder Vielfachen einer therapeutischen Einzeldosis entspricht. Eine Einzeldosis enthält vorzugsweise die Menge Wirkstoff, die bei einer Applikation verabreicht wird und die gewöhnlich einer ganzen, einer halben, einer drittel oder einer viertel Tagesdosis entspricht. Wenn für eine einzelne therapeutische Verabreichung nur ein Bruchteil, wie die Hälfte oder ein Viertel, der Einheitsdosis benötigt wird, ist die Einheitsdosis vorteilhafterweise teilbar, z.B. in Form einer Tablette mit Bruchkerbe.

Die pharmazeutischen Zubereitungen gemäß der Erfindung können, wenn sie in Einheitsdosen vorliegen und für die Applikation z.B. am Menschen bestimmt sind, etwa 10 bis 500 mg, vorteilhafterweise 50 bis 300 mg und insbesondere 100 bis 300 mg Wirkstoff enthalten. Parenterale Zubereitungen können etwa 2 bis 40 mg, vorteilhafterweise 4 bis 30 mg und insbesondere 10 bis 25 mg Wirkstoff enthalten.

Im allgemeinen hat es sich in der Humanmedizin als vorteilhaft erwiesen, den oder die Wirkstoffe bei oraler Gabe in einer Tagesdosis von 0,05 bis 5, vorzugsweise 0,5 bis 5, insbesondere 1 bis 3 mg/kg Körpergewicht, gegebenenfalls in Form mehrerer, vorzugsweise 1 bis 3 Einzelgaben zur Erzielung der gewünschten Ergebnisse zu verabreichen. Eine Einzelgabe enthält den oder die Wirkstoffe in Mengen von 0,05 bis 3, vorzugsweise 0,1 bis 3, insbesondere 0,5 bis 2 mg/kg Körpergewicht. Für die bevorzugte inhalative Verabreichung hat es sich als vorteilhaft erwiesen, den oder die Wirkstoffe in einer Tagesdosis von 0,5 bis 10 mg, vorzugsweise 1 bis 8 mg, insbesondere 2 bis 5 mg, gegebenenfalls in Form mehrerer, vorzugsweise 1 bis 3 Einzelgaben zu verabreichen.

Zubereitungen für die intravenöse Verabreichung sind vor allem für die Notfallbehandlung zweckmäßig.

Die therapeutische Verabreichung der pharmazeutischen Zubereitung kann 1 bis 4 mal am Tage zu festgelegten oder variierenden Zeitpunkten erfolgen, z.B. jeweils vor den Mahlzeiten und/oder am Abend. Es kann jedoch erforderlich sein, von den genannten Dosierungen abzuweichen, und zwar in Abhängigkeit von der Art, dem Körpergewicht und dem Alter des zu behandelnden Individuums, der Art und Schwere der Erkrankung, der Art der Zubereitung und der Applikation des Arzneimittels sowie dem Zeitraum bzw. Intervall, innerhalb welchem die Verabreichung erfolgt. So kann es in

einigen Fällen ausreichend sein, mit weniger als der oben genannten Menge Wirkstoff auszukommen, während in anderen Fällen die oben angeführte Wirkstoffmenge überschritten werden muß. Bei akuten Fällen wird zu Beginn der Behandlung eine höhere Dosis verabreicht. Nach Eintreten der gewünschten Wirkung wird auf eine niedrigere Dosis zurückgegangen.

Die Festlegung der jeweils erforderlichen optimalen Dosierung und Applikationsart der Wirkstoffe kann durch jeden Fachmann aufgrund seines Fachwissens leicht erfolgen.

Die pharmazeutischen Zubereitungen bestehen in der Regel aus den erfindungsgemäßen Wirkstoffen, gewünschtenfalls in Kombination mit Phosphodiesterasehemmern, und nichttoxischen, pharmazeutisch verträglichen Arzneimittelträgern, die als Zumischung oder Verdünnungsmittel in fester, halbfester oder flüssiger Form oder als Umhüllungsmittel, beispielsweise in Form einer Kapsel, eines Tablettenüberzugs, eines Beutels oder eines anderen Behältnisses, für den therapeutisch aktiven Bestandteil in Anwendung kommen. Ein Trägerstoff kann z.B. als Vermittler für die Arzneimittelaufnahme durch den Körper, als Formulierungshilfsmittel, als Süßungsmittel, als Geschmackskorrigens, als Farbstoff oder als Konservierungsmittel dienen.

Zur oralen Anwendung können z.B. Tabletten, Dragées, harte und weiche Kapseln, z.B. aus Gelatine, dispergierbare Pulver, Granulate, wäßrige und ölige Suspensionen, Emulsionen, Lösungen oder Sirupe kommen.

Tabletten können inerte Verdünnungsmittel, z.B. Calciumcarbonat, Calciumphosphat, Natriumphosphat oder Lactose; Granulierungs- und Verteilungsmittel, z.B. Maisstärke oder Alginate; Bindemittel, z.B. Stärke, Gelatine oder Akaziengummi; und Gleitmittel, z.B. Aluminium- oder Magnesiumstearat, Talkum oder Silikonöl, enthalten. Sie können zusätzlich mit einem Überzug versehen sein, der auch so beschaffen sein kann, daß er eine verzögerte Auflösung und Resorption des Arzneimittels im Gastrointestinaltrakt bewirkt, so daß z.B. eine bessere Verträglichkeit, Protrahierung oder eine Retardierung erreicht wird. Gelatinekapseln können den Arzneistoff vermischt mit einem festen, z.B. Calciumcarbonat oder Kaolin, oder einem öligen, z.B. Oliven-, Erdnuß- oder Paraffinöl, Verdünnungsmittel enthalten.

Wäßrige Suspensionen, die gegebenenfalls kurzfristig zubereitet werden, können Suspendiermittel, z.B. Natriumcarboxymethylcellulose, Methylcellulose, Hydroxypropylcellulose, Natriumalginat, Polyvinylpyrrolidon Traganthgummi oder Akaziengummi; Dispergier- und Benetzungsmittel, z.B. Polyoxyethylenstearat, Heptadecaethylenoxycetanol, Polyoxyethylensorbitolmonooleat, Polyoxyethylensorbitanmonooleat oder Lecithin; Konservierungsmittel, z.B. Methyl- oder Propylhydroxybenzoate; Geschmacksmittel; Süßungsmittel, z.B. Saccharose, Lactose, Natriumcyclamat, Dextrose, Invertzuckersirup, enthalten.

Ölige Suspensionen können z.B. Erdnuß-, Oliven-, Sesam-, Kokos- oder Paraffinöl und Verdickungsmittel, wie z.B. Bienenwachs, Hartparaffin oder Cetylalkohol, enthalten; ferner Süßungsmittel, Geschmacksmittel und Antioxidantien.

In Wasser dispergierbare Pulver und Granulate können die Arzneistoffe in Mischung mit Dispergier-, Benetzungs- und Suspendiermitteln, z.B. den oben genannten, sowie mit Süßungsmitteln, Geschmacksmitteln und Farbstoffen enthalten.

Emulsionen können z.B. Oliven-, Erdnuß- oder Paraffinöl neben Emulgiermitteln, wie z.B. Akaziengummi, Traganthgummi, Phosphatiden, Sorbitanmonooleat, Polyoxyethylensorbitanmonooleat, und Süßungs- und Geschmacksmitteln enthalten.

Zur rektalen Anwendung der Arzneistoffe werden Suppositorien verwendet, die mit Hilfe von bei Rektaltemperatur schmelzenden Bindemitteln, beispielsweise Kakaobutter oder Polyethylenglykol, hergestellt werden.

Zur parenteralen Anwendung der Arzneistoffe dienen steril injizierbare, gegebenenfalls kurzfristig herzustellende, wäßrige Suspensionen, isotonische Salzlösungen oder sonstige Lösungen, die Dispergier- oder Benetzungsmittel und/oder pharmakologisch verträgliche Verdünnungsmittel, z.B. Propylen- oder Butylenglykol, enthalten.

Die orale Anwendung der Arzneistoffe ist bevorzugt.

Bevorzugt ist auch die inhalative Applikation der erfindungsgemäßen Verbindungen. Diese werden entweder direkt als Pulver oder durch Vernebeln von die erfindungsgemäßen Verbindungen enthaltenden Lösungen oder Suspensionen verabreicht. Die Verneblung kann dabei auf herkömmliche Weise, beispielsweise durch Preßluftvernebler oder Ultraschallvernebler, erfolgen. Besonders vorteilhaft ist die Verabreichung aus Sprühdosen, insbesondere solcher mit einem herkömmlichen Dosierventil (Dosier-Aerosole). Mittels Dosier-Aerosolen ist es möglich, pro Sprühstoß eine definierte Menge an Wirkstoff bereitzustellen. Von besonderem Vorteil sind hier sogenannte Synchron-Inhalatoren, womit die Wirkstoffabgabe synchron zur Einatmung geschehen kann. Geeignete Synchron-Inhalationsvorrichtungen sind z.B. in der DE-PS 19 45 257, DE-PS 19 17 911 und DE-OS 20 55 734 offenbart.

Für Inhalationszwecke kommen die Wirkstoffe vorzugsweise in mikronisierter Form zum Einsatz, wobei Teilchengrößen von weniger als 10µm vorteilhaft sind. Zur Applikation aus Sprühdosen werden die Wirkstoffe in üblichen Treibmitteln dispergiert, vorzugsweise unter Zuhilfenahme eines Dispergiermittels. Als Treibmittel kommen insbesondere Gemische von Trichlorfluormethan (Frigen®11) und Dichlordifluormethan (Frigen®12) in Frage, wobei Trichlorfluormethan ganz oder teilweise durch 1,1,2-Trichlortrifluormethan (Frigen®113) ersetzt werden kann. Als Dispergiermittel kommen insbesondere die für diese Zwecke gebräuchlichen Sorbitanester (Spane® der Firma Atlas GmbH) und Lecithin in Frage. Das Dispergiermittel wird in der gekühlt vorgelegten schwerer flüchtigen Treibmittelkomponente gelöst. In die Lösung wird der mikronisierte Wirkstoff bzw. werden die mikronisierten Wirkstoffe eingerührt. Die Dispersion wird in Sprühbüchsen eingefüllt. Nach dem Vercrimpen wird die leichter flüchtige Treibmittelkomponente aufgedrückt.

Der oder die Wirkstoffe können gegebenenfalls mit einem oder mehreren der angegebenen Träger- oder Zusatzstoffe auch in mikroverkapselter Form formuliert werden.

Die erfindungsgemäßen Verbindungen zeigten am isolierten Meerschweinchen-Myokard positiv inotrope Wirkung bei nicht vorhandener positiv chronotroper oder sogar negativ chronotroper Wirkung; beides ist erwünscht für eine die Herzkraft selektiv steigernde Substanz.

Ferner wurde beobachtet, daß nach Verabreichung der erfindungsgemäßen Verbindungen eine Erschlaffung der glatten Muskulatur des Respirationstraktes eintrat, was für Substanzen für die Behandlung von Asthmaanfällen erwünscht ist. Schließlich macht die Wirkung der erfindungsgemäßen Verbindungen auf die glatte Muskulatur der Blutgefäße sie zur Regulation des Blutdrucks geeignet.

Die Erfindung wird in den folgenden Beispielen weiter veranschaulicht, ohne sie hierauf zu beschränken.

## Beispiele

1. 8-Brom-adenosin-3',5'-monophosphorsäurebenzylester (Verbindung 1)

a) 8-Bromadenosin-3',5'-cyclophosphorsäure

1,32 g (4,00 mMol) Adenosin-3',5'-cyclophosphorsäure (cAMP) werden in 2 ml 2n NaOH gelöst. Man verdünnt die sirupöse Lösung mit 25 ml eines 1n Natriumacetatpuffers (pH 3,9), gibt unter Rühren 880 mg (5,5 mMol) Brom in 35 ml des gleichen Puffers zu und rührt über Nacht. Anschliessend wird der entstandene Niederschlag abfiltriert, mit Wasser gewaschen, bis das Waschwasser farblos ist, dann mit Aceton und Ether nachgewaschen. Die 1,17 g (71 %) Rohprodukt werden zur weiteren Umsetzung verwendet.

UV-Spektrum (Wasser, pH 1): $\lambda_{max}$ (lg $\varepsilon$) = 263 nm (4,18)

b) 8-Bromadenosin-3',5'-monophosphorsäurebenzyltriester

816 mg (2,00 mMol) 8-Bromadenosin-3',5'-phosphorsäure werden in 20 ml Hexamethylphosphorsäuretriamid suspendiert (Ultraschallbad). Dazu wird eine etherische Lösung von Phenyldiazomethan, hergestellt aus 4,5 g N-Nitroso-N'-nitro-N-benzylguanidin (20 mMol), gegeben und bei Raumtemperatur unter Lichtausschluß gerührt. Wenn das Edukt in Lösung gegangen und seine Anwesenheit dünnschichtchromatographisch nicht mehr nachzuweisen ist, wird aufgearbeitet. Nach Abziehen des Lösungsmittels im Vakuum wird der Rückstand auf eine Kieselgelsäule gegeben, und die farbigen Nebenprodukte werden mit Methylenchlorid eluiert. Die Substanzzone wird mit Methylenchlorid/Methanol 10:1 bis 5:1 eluiert.

Ausbeute: 717 mg (72 %).

Das entstandene Diastereomerengemisch (ax:äq = 60:40) des axialen und äquatorialen Esters kann dünnschichtchromatographisch auf Kieselgel getrennt werden, Laufmittel ist Methylenchlorid/Methanol 10:1. Diese Trennung ist zwar zur Charkaterisierung der Einzelverbindungen angebracht, jedoch für die pharmakologische Verwendung nicht erforderlich. $Rf_{ax}$ = 0,60, $Rf_{äq}$ = 0,41; weitere charakterisierende Daten sind in der folgenden Tabelle I aufgeführt.

2. 8-Benzylthioadenosin-3',5'-monophosphorsäurebenzyltriester (Verbindung 3)

a) 8-Benzylthioadenosin-3',5'-phosphorsäure

408 mg (1,0 mMol) 8-Bromadenosin-3',5'-phosphorsäure werden in 20 ml Ethanol, das 0,1 mMol Natriumethylat enthält, unter Schutzgas ($N_2$) vorgelegt und dazu gleichzeitig 20 ml einer 0,25 n Natriumethylat-Lösung und 1,4 ml Benzylthiol gegeben. Anschließend wird 5 Stunden unter Rückfluß gekocht. Danach zieht man das Lösungsmittel ab, säuert mit 1n HCl an und entfernt überschüssiges Mercaptan im Vakuum. Eventuell gebildetes Disulfid wird ausgeethert. Der verbleibende Rückstand wird in 1n Ammoniak gelöst, nochmals mit Ether extrahiert und das Produkt durch Einstellen des pH auf 1 mit 2n HCl gefällt. Ausbeute 250 mg (58%). UV-Spektrum ($H_2O$, pH 1): $\lambda_{max}$ (log $\varepsilon$) 282 (4,30)

b) 8-Benzylthioadenosin-3',5'-monophosphorsäurebenzyltriester

300 mg (0,66 mMol) 8-Benzylthio-cAMP werden in 15 ml trockenem Aceton suspendiert, dazu gibt man eine etherische Phenyldiazomethanlösung (aus 3,2 g N-Nitroso-N'-nitro-N-benzylguanidin). Durch mehrmaliges Einengen und Zugeben von Aceton entfernt man den Ether möglichst vollständig. Die rote Lösung wird im Dunkeln bei Raumtemperatur 2 Tage gerührt und dann durch Abziehen des Lösungsmittels, Chromatographie auf einer Kieselgelsäule mit Methylenchlorid als Eluens aufgearbeitet. Die Substanz wird mit Methylenchlorid/Methanol 6:1 eluiert. Ausbeute 220 mg (61 %), Diastereomerengemisch. Die Ausbeute bei der Verwendung von Hexamethylphosphorsäuretriamid als Lösungsmittel beträgt 74 %.

Für die Charakterisierung der Substanz wurde das Diastereomerengemisch dünnschichtchromatographisch getrennt, was für die pharmakologische Aktivität (da additiv) jedoch nicht vonnöten ist.

Laufmittel $CH_2Cl_2$/MeOH 10:1. Verhältnis axial: äquatorial 54:46. $R_f$-Werte in Methylenchlorid/Methanol (10:1): $Rf_{ax}$ = 0,74; $Rf_{äq}$ = 0,61.

Weitere physikalische Daten finden sich in der folgenden Tabelle I, die auch zusätzliche beispielhaft hergestellte erfindungsgemäße Verbindungen und deren physikalische Daten enthält. Dabei bedeutet P den Verteilungskoeffizienten Octanol/Puffer, pH 7,4: $P = \frac{[\text{Substanz}] \text{ in Octanol}}{[\text{Substanz}] \text{ in Puffer, pH 7,4}}$

Tabelle I

Erfindungsgemäße Verbindungen und deren Charakterisierungsdaten

| Verb. Nr. | Substituenten $R^2 = C_6H_5$<br>$R^1$ = | Konfig. | UV (MeOH) $\lambda_{max}$ | UV (MeOH) ε | lg P | lg P Gemisch | Elementaranalyse, %, ber.<br>ax. gef.<br>äq. gef. | C | H | N | P |
|---|---|---|---|---|---|---|---|---|---|---|---|
| 1 | Br | ax<br>äq | 262<br>262 | 4,20<br>4,20 | 1,61<br>1,54 | 1,58 | $C_{17}H_{17}N_5O_6PBr \cdot 0,5C_4H_8O_2$<br>(MG 542,29) | 42,09<br>42,45<br>42,05 | 3,90<br>4,04<br>3,85 | 12,91<br>13,09<br>12,93 | 5,73<br>5,93<br>5,53 |
| 2 | $SCH_3$ | ax<br>äq | 278<br>278 | 4,26<br>4,23 | 1,55<br>1,70 | 1,62 | $C_{18}H_{20}N_5O_6PS \cdot 0,5C_4H_8O_2$<br>(MG 509,48) | 47,15<br>46,93<br>46,65 | 4,75<br>4,75<br>4,64 | 13,75<br>13,59<br>13,62 | 6,08<br>5,85<br>6,26 |
| 3 | $SCH_2C_6H_5$ | ax<br>äq | 282<br>282 | 4,26<br>4,26 | 1,96<br>1,88 | 1,92 | $C_{24}H_{24}N_5O_6PS \cdot 0,5C_4H_8O_2$<br>(MG 585,55) | 53,34<br>53,15<br>53,69 | 4,32<br>4,64<br>4,61 | 11,96<br>12,10<br>12,19 | 5,29<br>5,28<br>5,21 |
| 4 | $SeCH_2C_6H_5$ | ax<br>äq | 282<br>282 | 4,14<br>4,08 | 1,70<br>1,64 | 1,67 | $C_{24}H_{24}N_5O_6PSe \cdot 0,5C_4H_8O_2$<br>(MG 632,43) | 49,38<br>49,41<br>49,86 | 4,46<br>4,42<br>4,40 | 11,08<br>11,26<br>11,06 | 4,90<br>4,93<br>4,98 |
| 5 | $SC_6H_4$-4-Cl | ax<br>äq | 281<br>282 | 4,08<br>4,07 | | 2,24 | $C_{23}H_{21}N_5ClO_6PS \cdot 0,5C_4H_8O_2$<br>(MG 605,99) | 49,54<br>49,21<br>49,30 | 4,15<br>4,02<br>3,98 | 11,56<br>11,70<br>11,96 | 5,11<br>5,30<br>5,46 |
| 6 | $NHCH_2C_6H_5$ | ax<br>äq | 273<br>273 | 4,22<br>4,22 | | 1,97 | $C_{24}H_{25}N_6O_6P \cdot 0,5C_4H_8O_2$<br>(MG 568,53) | 54,90<br>54,43<br>54,50 | 5,14<br>5,40<br>5,45 | 14,78<br>15,44<br>15,54 | 5,45<br>5,20<br>5,11 |
| 7 | $NCH_3CH_2C_6H_5$ | ax<br>äq | 272<br>272 | 4,29<br>4,28 | | 2,29 | | | | | |

Vergleichsverbindungen: 1) Dibutyryl-cAMP = -1,10; 2) 8-Benzylthio-cAMP = -1,15; 3) cAMP-$OCH_2C_6H_5$ = 1,1 (lg P - Werte)

Tabelle I (Fortsetzung)
(Kernresonanzdaten)

| Verbindung | Konfig. | H-2 | aromat. Protonen | 6-$NH_2$ | H-1' | 2'-OH | P-O-$CH_2$-Ph (Dublett)[β] |
|---|---|---|---|---|---|---|---|
| 1 | ax[α] | 8.04 | 7.43 (M) | 6.41 | 6.02 | 5.6 (M) | 5.20 (8Hz) |
| | äq | 8.14 | 7.32 | 6.60 | 6.04 | 5.7 (M) | 5.20 (9Hz) |
| 2 | ax | 8.02 | 7.38 | 5.92 | 5.96 | 5.6 (M) | 5.20 (8Hz) |
| | äq[α] | 8.17 | 7.41 | 6.29 | 5.96 | 5.6 (M) | 5.17 (8Hz) |
| 3 | ax | 8.04 | 7.33 (M) | 5.82 | 5.97 | 5.5 (M) | 5.17 (9Hz) |
| | äq | 8.12 | 7.33 (M) | 6.05 | 5.99 | 5.6 (M) | 5.12 (9Hz) |
| 4 | ax | 8.03 | 7.36 (5H)<br>7.24 (5H) | 5.76 | 5.93 | 5.6 (M) | 5.18 (8Hz) |
| | äq[α] | 8.20 | 7.40 (5H)<br>7.29 (5H) | 6.16 | 5.95 | 5.6 (M) | 5.18 (10Hz) |

Die $^1$H-NMR-Spektren wurden mit einem 100 MHz-Spektrometer aufgenommen, die chemischen Verschiebungen sind in ppm gegen TMS ($\delta = 0$), die relative Anzahl der Protonen in Klammern angegeben. S = Singulett, D = Dublett, M = Multiplett

[α] Lösungsmittel: $CDCl_3$ + 1 Tropfen Methanol-$d_4$

[β] Dublett (Kopplungskonstante mit $^{31}P$)

Tabelle I (Fortsetzung Kernresonanzdaten)

| Verbindung | Konfig. | H-2' (Dublett)* | H-3' H-4' H-5' H-5" (S-$CH_2$-Ph) ** | -S-$CH_2$-Ph | S-$CH_3$ |
|---|---|---|---|---|---|
| 1 | ax[α] | 4.94 (6 Hz) | 4.20 - 4.76 (M) | — | — |
| | äq | 4.99 (5 Hz) | 4.20 - 4.67 (M) | — | — |
| 2 | ax | 4.99 (6 Hz) | 4.20 - 4.60 (M) | — | 2.72 |
| | äq[α] | 5.01 (4 Hz) | 4.20 - 4.65 (M) | — | 2.73 |
| 3 | ax | 4.89 (5 Hz) | 4.10 - 4.60 (M) | 4.48 | — |
| | äq | 5.02 (4 Hz) | 4.20 - 4.56 (M) | 4.48 | — |
| 4 | ax | 4.86 (6 Hz) | 4.10 - 4.76 (M) | 4.47 | — |
| | äq[α] | 4.89 (6 Hz) | 4.20 - 4.65 (M) | 4.49 | — |

* Dublett (Kopplungskonstante mit H-3')

** H-3', H-4', H-5', H-5" bilden eine komplizierte Multiplettstruktur; deswegen ist hier keine genaue Zuordnung möglich. Bei den Substanzen 3 und 4 liegen in diesem Gebiet außerdem die Benzylprotonen des 8-Substituenten

Tabelle I (Fortsetzung)
(Kernresonanzdaten)

| Verbindung | Konfig. | H-2 (s) | aromat. Protonen (m) | 6-$NH_2$ | H-1' (s) | H-2' (d) | P-$OCH_2$-$C_6H_5$ (d) | H-3',4',-5',5" (m) | -NH-$CH_2$-$C_6H_5$ (s) | $CH_3$-N-(s) |
|---|---|---|---|---|---|---|---|---|---|---|
| 5 *) | ax | 8,09 | 7,45 | - | 6,24 | 4,91 | 5,22 | 4,21-4,72 | | |
| | äq | 8,23 | 7,47 | - | 6,21 | 5,08 | 5,20 | 4,22-4,66 | | |
| 6 *) | ax | 7,89 | 7,39 | - | 5,83 | 4,92 | 5,20 | 4,10-4,60 | 4,65 | |
| | äq | 8,05 | 7,41 | - | 5,82 | 5,02 | 5,17 | 4,12-4,78 | 4,64 | |
| 7 | ax | 8,02 | 7,20 | 5,70 | 6,04 | 4,95 | 5,24 | 4,20-4,77 | 4,45 | 2,87 |
| | äq | 8,13 | 7,42 | 5,90 | 6,04 | 5,05 | 5,15 | 4,30-4,50 | 4,44 | 2,85 |

*) in $CDCl_3$/$CD_3OD$ aufgenommen

3. Tabletten mit 100 mg 8-(4-Chlorphenylthio)-3',5'-monophosphorsäurebenzyltriester

40,000 kg Wirkstoff, 24,000 kg Milchzucher und 16,000 kg Maisstärke werden mit 4,000 kg Polyvinylpyrrolidon (MG 25.000) in ungefähr 5,5 Liter Wasser granuliert und durch ein Sieb von 1,25 mm Maschenweite gepreßt. Nach dem Trocknen werden 10,000 kg Carboxymethylcellulose, 4,000 kg Talkum und 2,000 kg Magnesiumstearat zugegeben. Auf einer Exzentermaschine wird das Granulat zu Tabletten von 9 mm Durchmesser, 250 mg Gewicht und einer Härte von 4 bis 5 kg verpreßt.

4. Dosieraerosolzubereitung enthaltend 8-Brom-adenosin-3',5'-monophosphorsäurebenzylester

0,540 g Span® 85 und 0,135 g Aroma werden in 10,215 g gekühltem Frigen® 11 gelöst. In die Lösung werden 0,270 g mikronisierter Wirkstoff eingerührt und in 24 ml-Dosen eingefüllt. Nach dem Vercrimpen werden 14,971 g Frigen® 12 eingepreßt. Bei einem Kammervolumen des Dosierventils von 125 µl werden pro Ventilhub 1,6 mg 8-Brom-adenosin-3',5'-monophosphorsäurebenzylester als Aerosol freigesetzt.

Pharmakologie

Zur Bestimmung positiv inotroper Wirkungen wurden Papillarmuskeln aus dem rechten Ventrikel des Meerschweinchenherzens verwendet. Die adrenergen Nervenendigungen waren frei von Noradrenalin (Katecholamin-Entspeicherung durch intraperitoneale Injektion von 5 mg/kg Reserpin 24 Stunden vor Präparation der Tiere). Die Aufhängung der Muskeln erfolgte an einem induktiven Kraftaufnehmer in einer mit Sauerstoff gesättigten physiologischen Nährlösung (Volumen 50 ml). Die Kraftregistrierung erfolgte isometrisch, Kontraktionskurven wurden mittels eines Tintenschreibers aufgezeichnet und dann analysiert. Vor Zugabe der Testsubstanz wurden die Muskeln bei konstanter Reizung (Frequenz 0,2 Hz, Reizdauer 1 ms) bis zum Erreichen eines stationären Zustands ("steady state") der Kraft in der Nährlösung äquilibriert. Die Konzentration der Testsubstanz wurde dann erhöht, wenn die Kraft innerhalb von 10 min nicht weiter zunahm.

In der folgenden Tabelle II sind die Konzentrationen der einzelnen Testsubstanzen aufgetragen, bei denen der positiv inotrope Effekt gerade erkennbar war (Schwellenkonzentration) oder 50 % der maximalen Kraftzunahme erreichte ($EC_{50}$). Diese Werte wurden im unteren Teil der Tabelle denen bereits bekannter Testsubstanzen gegenübergestellt. Weiterhin werden Schwellenkonzentrationen und $EC_{50}$-Werte für die aufgeführten Testsubstanzen in Anwesenheit des Phosphodiesterase-Hemmstoffs 1-Methyl-3-isobutylxanthin (IBMX) (2 x $10^{-5}$ Mol/l) angegeben. Es resultiert eine Verschiebung zu 30 - 40-fach niedrigeren Konzentrationen im Vergleich zu den Werten ohne Phophodiesterase-Hemmstoff. Ein ähnlicher Synergismus wurde auch mit Papaverin (1 x $10^{-5}$ Mol/l) und mit Theophyllin (3 x $10^{-4}$ Mol/l) beobachtet.

**Tabelle II**

**Schwellenkonzentrationen von erfindungsgemäßen und Vergleichsverbindungen**

| Verbindung | Mol/l $EC_{50}$ | + 20 µMol IBMX $EC_{50}$ | Mol/l Schwellenkonz. | + 20 µMol IBMX Schwellenkonz. |
|---|---|---|---|---|
| 1 | $1 \times 10^{-5}$ | $1,5 \times 10^{-6}$ | $3 \times 10^{-6}$ | $3 \times 10^{-7}$ |
| 2 | $1 \times 10^{-5}$ | $4,5 \times 10^{-7}$ | $3 \times 10^{-6}$ | $1 \times 10^{-7}$ |
| 3 | $2 \times 10^{-5}$ | $6 \times 10^{-7}$ | $1 \times 10^{-5}$ | $1 \times 10^{-7}$ |
| 4 | $1 \times 10^{-5}$ | $5,5 \times 10^{-7}$ | $3 \times 10^{-6}$ | $1 \times 10^{-7}$ |
| 5 | $2 \times 10^{-6}$ | $3 \times 10^{-7}$ | $1 \times 10^{-7}$ | $3 \times 10^{-8}$ |
| 6 | $4 \times 10^{-5}$ | $2 \times 10^{-5}$ | $1 \times 10^{-5}$ | $5 \times 10^{-6}$ |
| 7 | | | | |
| Vergleichsverbindung | | | | |
| 1 | $1 \times 10^{-3}$ | $3 \times 10^{-4}$ | $3 \times 10^{-4}$ | $1 \times 10^{-4}$ |
| 2 | $8 \times 10^{-4}$ | $1,5 \times 10^{-4}$ | $1 \times 10^{-4}$ | $1 \times 10^{-5}$ |
| 3 | $2,5 \times 10^{-4}$ | $6 \times 10^{-5}$ | $1 \times 10^{-4}$ | $1 \times 10^{-5}$ |

Zur Bestimmung der erschlaffenden Wirkung von 8-$CH_3S$-cAMP-$OCH_2C_6H_5$ (Verbindung 2) auf die glatte Muskulatur des Respirationstraktes wurden isolierte Trachealstreifen des Meerschweinchens verwendet. Aufhängung der Präparate erfolgte wie bereits beschrieben. Nach Vorgabe einer geeigneten Testspannung (0,8 g) wurde die erschlaffende Wirkung der Testsubstanz in Abhängigkeit von der Konzentration getestet. Nach Auswaschen der Testsubstanz wurde der Versuch mit einem bereits bekannten cAMP-Derivat (Dibutyryl-cAMP, Vergleichsverbindung 1) wiederholt.

Tabelle III

Abnahme der Muskelspannung (T) in % des Kontrollwertes

| | | | | |
|---|---|---|---|---|
| Verbindung 2, Mol/l | $1 \times 10^{-6}$ | $3 \times 10^{-6}$ | $1 \times 10^{-5}$ | $3 \times 10^{-5}$ |
| T | 20 % | 85 % | 100 % | 100 % |
| Vergleichsverbindung 1 Mol/l | $1 \times 10^{-4}$ | $3 \times 10^{-4}$ | $1 \times 10^{-3}$ | $3 \times 10^{-3}$ |
| T | 8 % | 16 % | 75 % | nicht getestet |

Weiterhin wurde die relaxierende Wirkung erfindungsgemäßer Verbindungen auf die Trachealspangen-Kette des Meerschweinchens in vitro geprüft:

Vier parallele, aus jeweils 6 Einzelringen bestehende Trachealspangen-Ketten des Meerschweinchens (♂ und ♀, 430 - 600 g) im Organbad (5 ml, Krebs-Henseleit-Lösung mit Zusatz von Phentolamin ($10^{-5}$ Mol/l, 37°C, Vorspannung der Organe 2 g, Begasung mit Carbogen) entwickeln nach etwa 20 bis 30 Minuten eine stabile, tonische Spontankontraktur. An diesen dauerkontrahierten Organen kann unter isometrischen Meßbedingungen durch Applikation der Prüfsubstanz in kumulativhalblogarithmisch ansteigender Konzentration (z.B. $1x10^{-6}$ + $2x$-$10^{-6}$ + $7x10^{-6}$ + $2x10^{-5}$ usw. Mol/l) eine Relaxation herbeigeführt werden, wobei nach jeder Einzeldosis der Testsubstanz eine konstante Relaxations-Antwort abgewartet wird, bevor die nächst höhere Konzentration appliziert wird. Über einen Zeitraum von 20

bis 30 Minuten wird somit eine vollständige Dosis-Wirkungskurve der Testsubstanz erhalten. Die jeweilige Relaxation wird als Prozentbruchteil der durch Gabe von (-)Isoprenalin ($10^{-6}$ Mol/l) maximal erreichbaren Relaxation ausgedrückt.

In Tabelle IV sind die $ED_{50}$ (Dosis der halbmaximal möglichen Relaxation) und die relative Wirkungsstärke im Vergleich zu Theophyllin für erfindungsgemäße Verbindungen angegeben.

Tabelle IV

| Verbindung Nr. | $ED_{50}$ [Mol/l] | Relative Wirkungsstärke (Theophyllin = 1,00) |
|---|---|---|
| 1 | $7 \times 10^{-6}$ | 18,5 |
| 5 | $3,8 \times 10^{-5}$ | 3,4 |
| Theophyllin | $1,3 \times 10^{-4}$ | 1,00 |

Patentansprüche

1. Substituierte Adenosin-3',5'-phosphorsäurebenzyltriester der allgemeinen Formel I

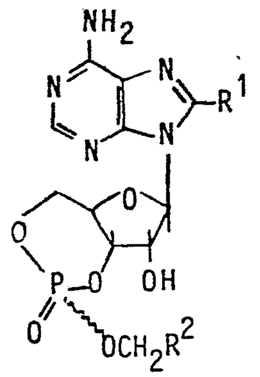

I,

wobei

$R^1$ die Bedeutung Halogen, $SR^3$, worin $R^3$ einen geradkettigen oder verzweigten $C_1$-$C_5$-Alkyl- oder $C_2$-$C_5$-Alkenylrest, die durch Hydroxy oder Phenyl, das mit Halogen, Methyl, Ethyl, Methoxy, Ethoxy oder Nitro substituiert sein kann, substituiert sein können, einen $C_4$-$C_7$-Cycloalkylrest, Phenyl oder Naphthyl, die mit Halogen, Methyl, Ethyl, Methoxy oder Ethoxy ringsubstituiert sein können, bedeutet, $SeR^4$, worin $R^4$ einen geradkettigen oder verzweigten $C_1$-$C_5$-Alkylrest, oder Benzyl, das mit Halogen, Methyl, Ethyl, Methoxy, Ethoxy oder Nitro ringsubstituiert sein kann, bedeutet, oder $NR^5R^6$, worin $R^5$ und $R^6$, die gleich oder verschieden sind, Wasserstoff oder $R^3$ bedeuten, oder zusammen mit dem Stickstoffatom einen Piperidino oder Morpholinorest darstellen, und

$R^2$ die Bedeutung Phenyl, das durch elektronenliefernde oder elektronenziehende Gruppen substituiert sein kann, haben.

2. Substituierte Adenosin-3',5'-phosphorsäurebenzyltriester der allgemeinen Formel I'

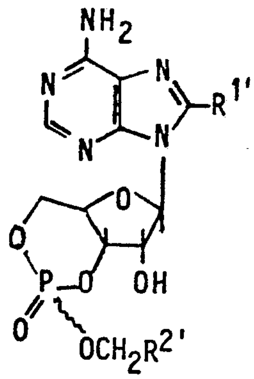

I',

wobei

$R^{1'}$ die Bedeutung Chlor, Brom, Iod, Methylthio, Ethylthio, t-Butylthio, Cyclohexylthio, Benzylthio, wobei die Phenylgruppe durch Halogen, Methyl oder Ethyl monosubstituiert sein kann, Phenylthio, wobei die Phenylgruppe durch Halogen, Methyl oder Ethyl monosubstituiert sein kann, Benzylseleno, wobei die Phenylgruppe durch Halogen, Methyl oder Ethyl monosubstituiert sein kann, $NR^{5'}R^{6'}$, wobei $R^{5'}$, $R^{6'}$ gleich oder verschieden sind und Wasserstoff, Methyl, Ethyl oder Benzyl bedeuten, und

$R^{2'}$ die Bedeutung Phenyl, wobei die Phenylgruppe durch Halogen, Methyl, Methoxy, Cyan oder Nitro einfach oder mehrfach substituiert sein kann, haben.

3. Substituierte Adenosin-3',5'-phosphorsäurebenzyltriester der allgemeinen Formel I"

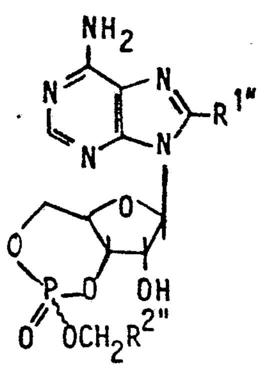

I",

wobei

$R^{1''}$ die Bedeutung Brom, Methylthio, t-Butylthio, Cyclohexylthio, Benzylthio, p-Chlorphenylthio, Benzylseleno, Benzylamino, Benzylmethylamino, und

$R^{2"}$ die Bedeutung Phenyl, p-Cyanophenyl und p-Methylphenyl haben.

4. Verfahren zur Herstellung einer Verbindung gemäß einem der Ansprüche 1 bis 3, dadurch gekennzeichnet, daß eine Verbindung der allgemeinen Formel 8-$R^1$-cAMP, 8-$R^{1'}$-cAMP bzw. 8-$R^{1"}$-cAMP, worin $R^1$, $R^{1'}$ bzw. $R^{1"}$ die in den Ansprüchen 1, 2 bzw. 3 angegebene Bedeutung hat und cAMP Adenosin-3',5'-monophosphorsäure bedeutet, mit einem Diazomethanderivat der allgemeinen Formel $R^2CHN_2$, $R^{2'}CHN_2$ bzw. $R^{2"}CHN_2$, worin $R^2$, $R^{2'}$ bzw. $R^{2"}$ die in den Ansprüchen 1, 2 bzw. 3 angegebene Bedeutung hat, verestert wird.

5. Verfahren nach Anspruch 4, dadurch gekennzeichnet, daß die Veresterung in Hexamethylphosphorsäuretriamid durchgeführt wird.

6. 8-Brom-adenosin-3',5'-monophosphorsäurebenzylester.

7. 8-(4-Chlorphenylthio)-adenosin-3',5'-monophosphorsäurebenzylester.

8. 8-Methylthio-adenosin-3',5'-monophosphorsäurebenzylester.

9. Arzneimittel enthaltend eine oder mehrere Verbindungen gemäß einem oder mehreren der Ansprüche 1 bis 3 und 6 bis 8.

10. Verbindungen gemäß einem oder mehreren der Ansprüche 1 bis 3 und 6 bis 8 zur Anwendung in der Behandlung von Erkrankungen des Herzens und/oder des Respirationstraktes.

11. Arzneimittel nach Anspruch 9, gekennzeichnet durch einen zusätzlichen Gehalt an einer die Phosphodiesterase hemmenden Substanz.

12. Arzneimittel nach Anspruch 11 gekennzeichnet durch den Gehalt an 1-Methyl-3-isobutylxanthin, Theophyllin und/oder Papaverin als Phosphodiesterase hemmende Substanz.

13. Arzneimittel nach einem der Ansprüche 9, 11 oder 12, dadurch gekennzeichnet, daß es als Dosieraerosolzubereitung vorliegt.

0044527

Europäisches Patentamt

**EUROPÄISCHER RECHERCHENBERICHT**

Nummer der Anmeldung
EP 81 10 5575

| EINSCHLÄGIGE DOKUMENTE | | | KLASSIFIKATION DER ANMELDUNG (Int. Cl.³) |
|---|---|---|---|
| Kategorie | Kennzeichnung des Dokuments mit Angabe, soweit erforderlich, der maßgeblichen Teile | betrifft Anspruch | |
| | JOURNAL OF MEDICINAL CHEMISTRY, Band 20, Nr. 7, Juli 1977, Seiten 907/910 KUO-HSIUNG LEE et al.: "Antitumor agents. 25. Synthesis and antitumor activity of uracil and thymine α-methylene-γ-lactones and related derivatives" * Seite 907 * -- | 1-13 | C 07 H 19/20<br>A 61 K 31/70 |
| | US - A - 3 712 885 (G. WEIMANN) * Anspruch 1 * -- | 1-13 | |
| | | | RECHERCHIERTE SACHGEBIETE (Int. Cl.³) |
| D | JOURNAL OF MEDICINAL CHEMISTRY, Band 23, Nr. 3, März 1980, Seiten 242-251 J.P. MILLER: "Synthesis and enzymatic and inotropic activity of some new 8-substituted and 6,8-disubstituted derivatives of adenosine cyclic 3',5'-monophosphate" * Seite 242 * ---- | 1-13 | C 07 H 19/20<br>A 61 K 31/70 |
| | | | KATEGORIE DER GENANNTEN DOKUMENTE<br>X: von besonderer Bedeutung<br>A: technologischer Hintergrund<br>O: nichtschriftliche Offenbarung<br>P: Zwischenliteratur<br>T: der Erfindung zugrunde liegende Theorien oder Grundsätze<br>E: kollidierende Anmeldung<br>D: in der Anmeldung angeführtes Dokument<br>L: aus andern Gründen angeführtes Dokument |
| | Der vorliegende Recherchenbericht wurde für alle Patentansprüche erstellt. | | &: Mitglied der gleichen Patentfamilie, übereinstimmendes Dokument |

| Recherchenort | Abschlußdatum der Recherche | Prüfer |
|---|---|---|
| Den Haag | 07-09-1981 | VERHULST |

EPA form 1503.1 06.78